(19) Europäisches Patentamt
European Patent Office
Office européen des brevets

(11) **EP 3 603 484 B1**

(12) **EUROPEAN PATENT SPECIFICATION**

(45) Date of publication and mention
of the grant of the patent:
**17.08.2022  Bulletin 2022/33**

(21) Application number: **18788415.0**

(22) Date of filing: **11.04.2018**

(51) International Patent Classification (IPC):
**A61B 3/10** *(2006.01)*        **A61B 90/20** *(2016.01)*
**A61F 9/007** *(2006.01)*

(52) Cooperative Patent Classification (CPC):
**A61B 3/10; A61B 3/102; A61B 90/20; A61F 9/007**

(86) International application number:
**PCT/JP2018/015228**

(87) International publication number:
**WO 2018/193932 (25.10.2018 Gazette 2018/43)**

(54) **INFORMATION PROCESSING DEVICE, SURGICAL TOOL, INFORMATION PROCESSING METHOD, AND PROGRAM**

INFORMATIONSVERARBEITUNGSVORRICHTUNG, CHIRURGISCHES WERKZEUG, INFORMATIONSVERARBEITUNGSVERFAHREN UND PROGRAMM

DISPOSITIF DE TRAITEMENT D'INFORMATIONS, INSTRUMENT CHIRURGICAL, PROCÉDÉ DE TRAITEMENT D'INFORMATIONS ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **21.04.2017   JP 2017084675**

(43) Date of publication of application:
**05.02.2020   Bulletin 2020/06**

(73) Proprietor: **Sony Group Corporation
Tokyo 108-0075 (JP)**

(72) Inventor: **SOMA, Yoshio
Tokyo 108-0075 (JP)**

(74) Representative: **D Young & Co LLP
120 Holborn
London EC1N 2DY (GB)**

(56) References cited:
WO-A1-2015/189227      JP-A- 2009 201 682
JP-A- 2016 073 409      US-A1- 2012 184 846
US-A1- 2014 221 822      US-A1- 2015 173 644

**Description**

Technical Field

The present technology relates to technologies such as an information processing apparatus that executes processing related to a tomographic image of an eye which is displayed during surgical operation for the eye.

Background Art

In recent years, surgical microscope apparatuses have been widely used in the surgical operation on the eye. This surgical microscope apparatus causes an image of the eye which is acquired via a microscope and a tomographic image of the eye which is acquired by an optical coherence tomography (OCT) or the like to be displayed. A user performs a surgical operation for the eye while referring to those images. With this configuration, generation of surgical errors is prevented and, in addition, surgical operation accuracy is improved.

The OCT is a technology of irradiating an eye with a near-infrared ray and reconstructing light reflected by respective tissues of the eye to generate an image. With the OCT, a tomographic image of the eye in a particular tomographic surface can be obtained. During surgical operation, not only the eye but also a surgical tool for performing a surgical operation on the eye are shown in the tomographic image.

The surgical tool is generally constituted by metal. Therefore, in the OCT, a near-infrared ray radiated to the eye is reflected by the surgical tool and the reflected light from the eye cannot be obtained below the surgical tool. Therefore, in the tomographic image, no signals are obtained below the surgical tool and a shadow appears. Further, since the surgical tool is constituted by metal, artifacts may be generated around the surgical tool in the tomographic image.

Non-Patent Literature 1 below has been disclosed as a technology associated with such a problem. In accordance with Non-Patent Literature 1, a distal end portion of a surgical tool is constituted by a resin material transmissive to near-infrared rays and generation of shadows and artifacts is suppressed in a tomographic image by using such a surgical tool, for example.

Citation List

Patent Literature

Non-Patent Literature 1: Ehlers, Justis P., et al. "Integrative advances for OCT-guided ophthalmic surgery and intraoperative OCT: microscope integration, surgical instrumentation, and heads-up display surgeon feedback." PLoS One 9.8 (2014): e105224.

Prior Art Includes: US 2014/221822 A1. This document discloses an ophthalmologic surgical instrument comprising optical markers which can be identified in OCT images. It also discloses a scan processor for detecting a relative position of the instrument in OCT images.

Disclosure of Invention

Technical Problem

However, in a case where the surgical tool is constituted by a resin, there is a problem in that the surgical tool is detected as a low signal in the tomographic image and it becomes difficult for a user to recognize a position or attitude of the surgical tool in the tomographic image.

In view of the above-mentioned circumstances, it is an object of the present technology to provide a technology by which even in a case where the surgical tool is constituted by a resin, the position or attitude of the surgical tool can be correctly detected in the tomographic image.

Solution to Problem

Embodiments of the present disclosure are defined by the appended Claims.

Advantageous Effects of Invention

As described above, in accordance with the present technology, it is possible to provide a technology by which even in a case where the surgical tool is constituted by a resin, the position or attitude of the surgical tool can be correctly detected in the tomographic image.

Brief Description of Drawings

[Fig. 1] A block diagram showing a configuration of a surgical microscope apparatus according to a first embodiment.
[Fig. 2] A schematic diagram showing a cataract surgery process.
[Fig. 3] A schematic diagram showing a cataract surgery process.
[Fig. 4] A diagram showing an example of a surgical tool.
[Fig. 5] An enlarged diagram showing a marker portion.
[Fig. 6] A net showing a marker portion.
[Fig. 7] A diagram showing a resin portion and a marker portion which are shown in a tomographic image in a case where an X'Z' plane indicated as A-A in Fig. 5 is set as a tomographic surface.
[Fig. 8] An enlarged diagram showing a marker portion.
[Fig. 9] A net showing a marker portion.
[Fig. 10] A diagram showing a resin portion and a marker portion which are shown in a tomographic image in a case where an X'Z' plane indicated as B-B in Fig. 8 is set as the tomographic surface.
[Fig. 11] A diagram showing a resin portion and a marker portion which are shown in a tomographic image in a case where a Y'Z' plane indicated as C-C in Fig. 8 is set as the tomographic surface.
[Fig. 12] A flowchart showing processing of the control unit.
[Fig. 13] A diagram showing an example of a front image acquired by a front image acquisition unit.
[Fig. 14] A diagram showing an example of a tomographic image acquired by a tomographic image acquisition unit.
[Fig. 15] A diagram showing a state when position or attitude information of the surgical tool is superimposed on the front image.
[Fig. 16] A diagram showing a state when the position or attitude information of the surgical tool is superimposed on the tomographic image.
[Fig. 17] A flowchart showing processing according to a second embodiment.
[Fig. 18] A diagram showing an example when a tomographic surface is input by a user.
[Fig. 19] A diagram showing a state when the surgical tool is deviated with respect to the tomographic surface.
[Fig. 20] A diagram showing a state when the surgical tool is deviated with respect to the tomographic surface.
[Fig. 21] A diagram showing a state when the surgical tool is deviated with respect to the tomographic surface.
[Fig. 22] A diagram showing an example of amount-of-deviation information presented to the user.
[Fig. 23] A diagram showing an example of amount-of-deviation information presented to the user.

Mode(s) for Carrying Out the Invention

Hereinafter, embodiments according to the present technology will be described with reference to the drawings.

<<First Embodiment>>

<Configuration of Surgical Microscope Apparatus>

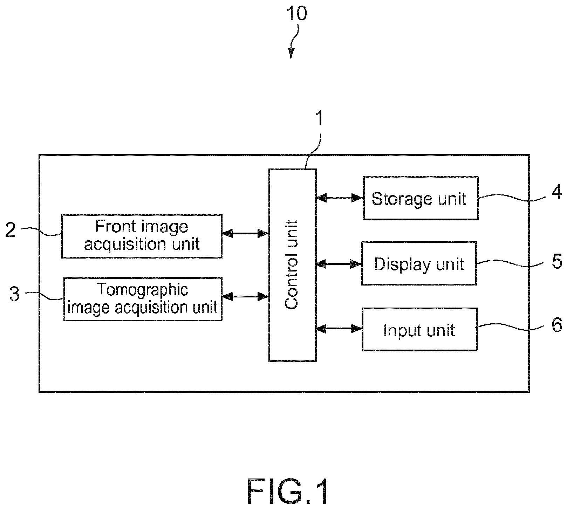

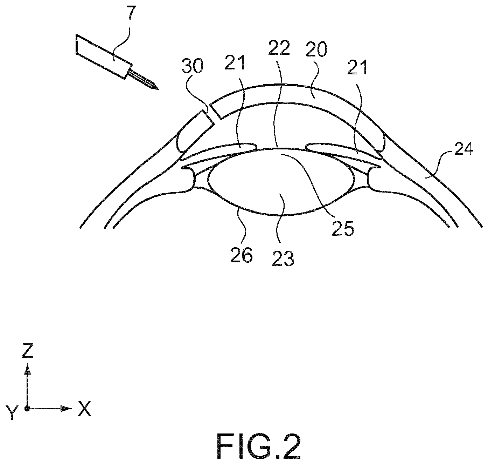

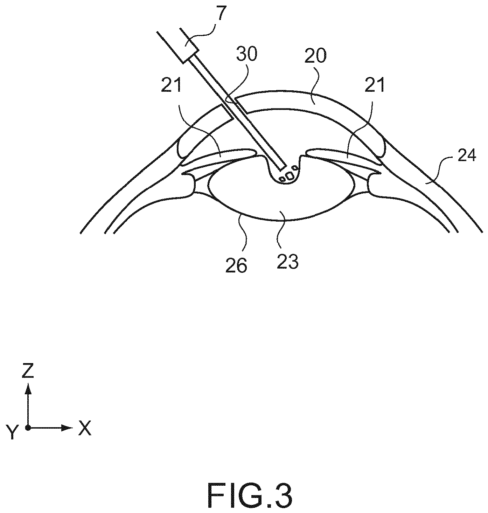

Fig. 1 is a block diagram showing a configuration of a surgical microscope apparatus 10 according to a first embodiment. As shown in Fig. 1, the surgical microscope apparatus 10 (information processing apparatus) includes a control unit 1, a front image acquisition unit 2, a tomographic image acquisition unit 3, a storage unit 4, a display unit 5, and an input unit 6.
It should be noted that in the description of this embodiment, it is assumed that a depth direction of an eye is a Z-axis direction and two orthogonal axes in a plane orthogonal to the Z-axis direction are an X-axis direction and a Y-axis direction (see Figs. 2 and 3, etc. to be described later).
The front image acquisition unit 2 includes, for example, a microscope apparatus with a camera, a microscope apparatus with a stereo camera, and the like. The front image acquisition unit 2 takes an image of an eye which is a surgical operation target from the front, acquires a front image, and outputs the acquired front image to the control unit 1.
The tomographic image acquisition unit 3 includes an OCT in this embodiment. It should be noted that the tomographic image acquisition unit 3 may include a Scheimpflug camera. The tomographic image acquisition unit 3 irradiates the eye with a near-infrared ray and utilizes light interference of reflected light from the eye and reference light to execute scan in a depth direction (Z-axis direction) of the eye.
The tomographic image is configured in such a manner that rectangular images very elongated in the depth direction (hereinafter, rectangular images) are arranged along an arbitrary direction in a plane direction (XY direction). Therefore, the tomographic image acquisition unit 3 is configured to be capable of not only scanning in the depth direction but also scanning in any one direction in at least the plane direction.

The tomographic image acquisition unit 3 acquires a tomographic image on the basis of various parameters. Here, the various parameters include a measurement position, a measurement region, a scan density, a tomographic surface, and the like.

The measurement position is a position in which the measurement by the OCT is performed. The measurement region is a region in the plane direction in which the measurement by the OCT is performed (a region in the plane direction in which scan is performed).

Here, the measurement region may be set to be linear (scan line) as the eye is viewed from above (Z-axis direction) or may be set to be planar as the eye is viewed from above. In a case where the measurement region is set to be planar, the volume data (three-dimensional data) of the eye can be obtained.

The measurement region is fixed in this embodiment. It should be noted that the measurement region may be set in accordance with a user's instruction. The scan density is density of scan in the plane direction (a value indicating at which intervals the measurement by the OCT is performed in the plane direction).

Further, the tomographic surface is a surface which is a criteria when generating a tomographic image. That is, the tomographic image is generated in such a manner that the rectangular images are arranged along a direction of the tomographic surface by using the tomographic surface as a reference. The tomographic surface may be specified by the user or may be determined on the basis of a position of a surgical tool 7 (see Figs. 2 and 3).

It should be noted that in a case where the measurement region is set to be linear (scan line), the direction of the tomographic surface is identical to a linear measurement region. In this case, only by simply arranging the acquired rectangular images, the tomographic image is generated. On the other hand, in a case where the measurement region is set to be planar, rectangular images of rectangular images acquired in the planar measurement region, which are located in a direction along the tomographic surface, are picked up and the picked up rectangular images are arranged. The tomographic image is thus generated.

The display unit 5 includes a liquid-crystal display, an organic electro luminescence (EL) display, and the like. The display unit 5 causes a front image of the eye acquired by the front image acquisition unit 2 and a tomographic image of the eye acquired by the tomographic image acquisition unit 3 to be displayed on the screen. It should be noted that in a case where the tomographic image acquisition unit 3 is capable of acquiring the volume data, not only the tomographic image of the eye but also a three-dimensional image of the eye may be displayed on the display unit 5.

The input unit 6 includes, for example, a keyboard, a mouse, a touch sensor provided on the screen of the display unit 5, and the like. The input unit 6 inputs a user's operation signal and outputs the input user's operation signal to the control unit 1.

The control unit 11 includes a central processing unit (CPU) and the like. The control unit 11 executes various types of calculation based on various programs stored in the storage unit 42 and comprehensively controls the respective units of the surgical microscope apparatus 10. It should be noted that processing of the control unit 11 will be described later in detail in the section of the operation description.

The storage unit 42 includes a nonvolatile memory in which various programs and various types of data required for the processing of the control unit 11 are stored and a volatile memory to be used as a working area for the control unit 11. It should be noted that various programs stored in the storage unit 42 may be read from a portable recording medium such as an optical disc and a semiconductor memory or may be downloaded from a server apparatus over a network.

<Outline of Ophthalmologic Surgery>

Next, the outline of the cataract surgery in which the surgical microscope apparatus 10 can be utilized will be described. Figs. 2 and 3 are schematic diagrams each showing a cataract surgery process. As shown in those figures, the eyeball includes tissues such as a cornea 20, an iris 21, a lens 23, and a sclera 24. An anterior capsule 25 is formed on a front surface in the lens 23. A posterior capsule 26 is formed on a rear surface in the lens 23. At the back of the lens 23, a vitreous body (not shown) is located. Further, it is a pupil 22 that is located in the middle of the iris 21 on the surface of the lens 23.

As shown in Fig. 2, in the cataract surgery, a surgical wound 30 is formed in the cornea 20 through a surgical tool 7 such as a scalpel. Next, the surgical tool 7 is inserted through the surgical wound 30 and the anterior capsule 25 in the lens 23 is incised through the surgical tool 7, an interior (core or cortex) of the lens 23 is absorbed and removed through a surgical tool 7 for absorption as shown in Fig. 3. After that, an intraocular lens is inserted at the position at which the lens 23 has been removed. The surgery is thus completed.

It should be noted that the cataract surgery shown here is an example of the ophthalmologic surgery in which the surgical microscope apparatus 10 can be utilized and the surgical microscope apparatus 10 can be utilized in various types of ophthalmologic surgery.

<Configuration of Surgical Tool 7>

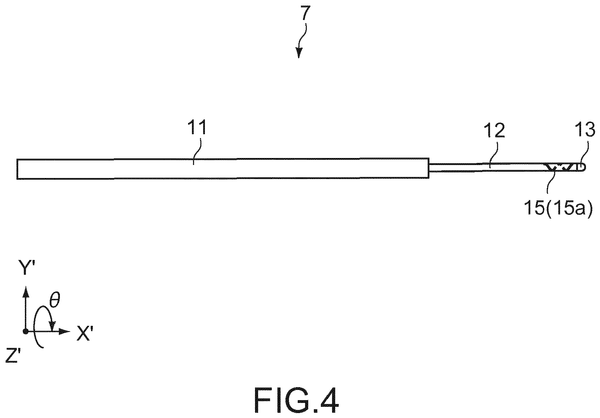

Next, a configuration of the surgical tool 7 will be described in detail. Fig. 4 is a diagram showing an example of the surgical tool 7. As shown in Fig. 4, the surgical tool 7 includes a shape long in the longitudinal direction.

It should be noted that in the description of the present specification, it is assumed that the longitudinal direction of the surgical tool 7 is an X' axis direction and two orthogonal axes are a Y' axis direction and a Z' axis direction in a plane orthogonal to the longitudinal direction of the surgical tool 7. Further, it is assumed that a direction around an X' axis is a θ direction.

The surgical tool 7 includes a gripping portion 11 to be gripped by the user, a resin portion 12 provided on the gripping portion 11 and positioned on a side of the distal end of the surgical tool 7, and a surgical operation portion 13 provided on the resin portion 12 and positioned at the distal end of the surgical tool 7.

The gripping portion 11 is typically constituted by a metal material. Alternatively, the gripping portion 11 may be constituted by a material such as a resin. The material of the gripping portion 11 is not particularly limited. The gripping portion 11 is set to have size and shape easy for the user to hold it in hand.

The surgical operation portion 13 is typically constituted by metal. Alternatively, the surgical operation portion 13 may be constituted by a material such as a resin. The material of the gripping portion 11 is not particularly limited. The surgical operation portion 13 is a portion that performs a surgical operation such as incision, pealing, absorption, removal, and the like on each of tissues constituting the eye. This surgical operation portion 13 is configured in various shapes such as a scalpel shape, a forceps shape, cylindrical shape, and the like, for example.

The resin portion 12 includes a resin material transmissive to near-infrared rays to be used in the OCT. In this embodiment, the resin portion 12 includes a columnar shape long in the longitudinal direction. It should be noted that the shape of the resin portion 12 may have a prism shape or the like. The shape is not particularly limited. Further, the resin portion 12 may be arranged at plurality of positions at predetermined intervals along the longitudinal direction.

In this embodiment, since the resin portion 12 is constituted by the resin material transmissive to near-infrared rays to be used in the OCT, generation of shadows and artifacts in the tomographic image can be prevented.

On the other hand, if any measures are not applied in the resin portion 12, the resin portion 12 in the surgical tool 7 can be detected as a low signal in the tomographic image and it can be difficult for the user to recognize the position or attitude of the surgical tool 7 in the tomographic image. It can cause surgical errors. Further, in a case where image recognition is performed on the basis of the tomographic image, a contrast between the resin portion 12 in the surgical tool 7 and the background portion (eye) becomes smaller, and thus it becomes difficult to detect a region of the surgical tool 7.

In view of this, in this embodiment, a marker portion 15 having a particular geometric pattern is arranged in the resin portion 12. The marker portion 15 includes a material detectable as a higher signal than the resin portion 12 in acquisition of the tomographic image in the OCT.

For example, the marker portion 15 includes a mixture of metal, resin, and metal, a coating, and the like. Since the marker portion 15 is constituted by a material detectable as a higher signal than the resin portion 12, a position of the marker portion 15 in the tomographic image can be correctly detected.

The position of the marker portion 15 is adjusted such that the marker portion 15 is shown in a tomographic screen during surgical operation. Hereinafter, a configuration of the marker portion 15 will be described in detail.

[Configuration (1) of Marker Portion 15]

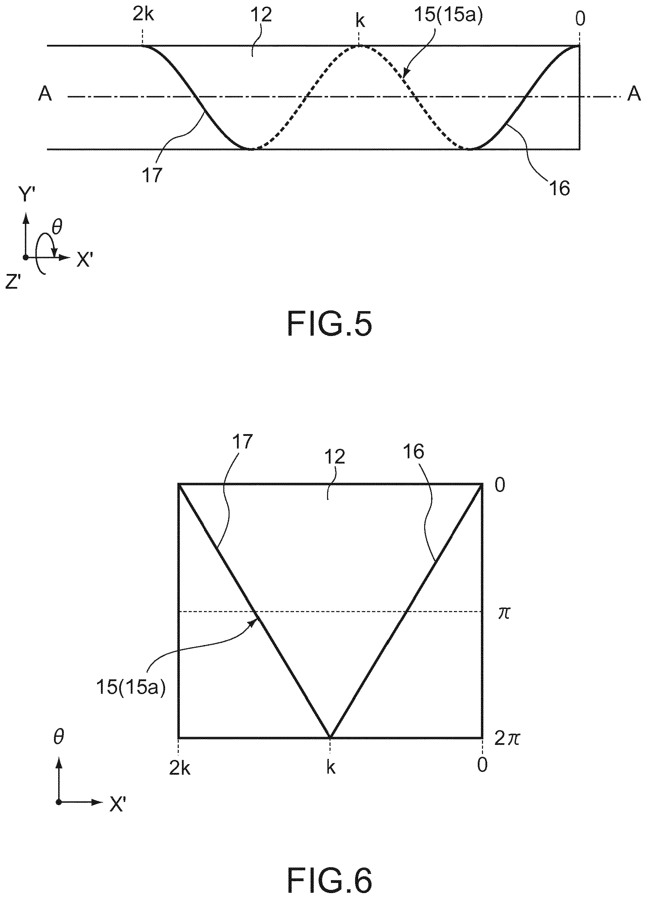

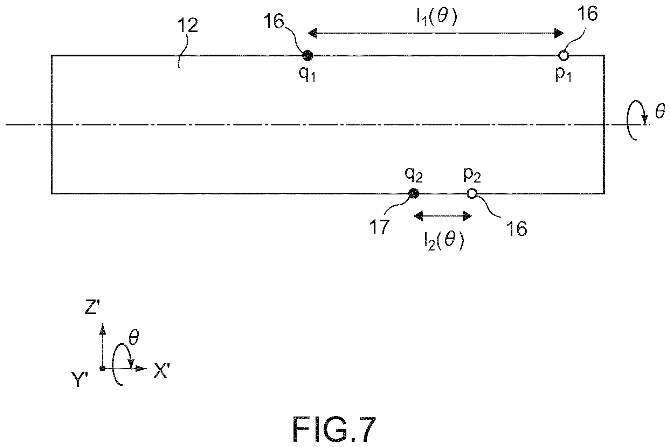

Fig. 5 is an enlarged diagram showing a marker portion 15a. Fig. 6 is a net showing a marker portion 15a. It should be noted that in Figs. 5 and 6, a right-hand side is a distal-end side (the same applies to Fig. 7 to be described later). Further, in Fig. 6, the vertical direction is the θ direction (the direction around the X' axis) and the horizontal direction is the X' axis direction (the longitudinal direction).

As shown in those figures, the marker portion 15a includes a first marker 16 and a second marker 17. The first marker 16 is wound once around the resin portion 12 in a particular direction in the θ direction in a spiral form. Further, the second marker 17 is wound once around the resin portion 12 in a direction opposite to the particular direction in the θ direction in a spiral form.

It should be noted that a distance in the X' axis direction from a foremost end position (position of the first marker 16 which is closest to the distal end of the surgical tool 7) to a rearmost end position (position of the first marker 16 which is closest to the rear end of the surgical tool 7) of the first marker 16 and a distance in the X' axis direction from a foremost end position (position of the first marker 17 which is closest to the distal end of the surgical tool 7) to a rearmost end position (position of the first marker 17 which is closest to the rear end of the surgical tool 7) of the second marker 17 are each equally set to k.

Fig. 7 is a diagram showing the resin portion 12 and the marker portion 15a shown in the tomographic image in a case where an X'Z' plane indicated as A-A in Fig. 5 is set as the tomographic surface.

In the tomographic image in Fig. 7, the first marker 16 wound around the resin portion 12 cross at a total of two points, at a single point of each of the upper and lower sides of the resin portion 12 with respect to the X'Z' plane. It is assumed that the upper point at this time is $p_1(\theta)$ and the lower point is $p_2(\theta)$.

Similarly, the second marker 17 wound around the resin portion 12 crosses at a total of two points, at a single point of each of the upper and lower sides of the resin portion 12 with respect to the X'Z' plane. It is assumed that the upper point at this time is $q_1(\theta)$ and the lower point is $q_2(\theta)$.

It should be noted that in the X' axis direction, the foremost end position of the first marker 16 is set as a point-of-origin (i.e., 0) and a direction toward the back side from this point-of-origin is set as a positive direction. Further, the value of $\theta$ is set to be 0 when the foremost end position in the first marker 16 and the rearmost end position in the second marker 17 cross the X'Z' plane and the angle around the X' axis in the resin portion 12 is set to use the angle at this time as a criteria. Further, a clockwise direction as the resin portion 12 is viewed from the distal-end side is set as a positive direction in the $\theta$ direction.

At this time, the respective points of $p_1(\theta)$, $p_2(\theta)$, $q_1(\theta)$, and $q_2(\theta)$ are respectively expressed in accordance with the following expressions. Where the value of k is a distance in the X' axis direction from the foremost end position to the rearmost end position of the first marker 16 and is also a distance in the X' axis direction from the foremost end position to the rearmost end position of the second marker 17.

$$p_1(\theta) = k\theta/2\pi$$

$$p_2(\theta) = k(\theta + \pi)/2\pi \ldots \quad (0 \leq \theta < \pi)$$

$$= k(\theta-\pi)/2\pi \ldots \quad (\pi \leq \theta < 2\pi)$$

$$q_1(\theta) = -k\theta/2\pi + 2k$$

$$q_2(\theta) = k(\theta + \pi)/2\pi + 2k \ldots \quad (0 \leq \theta < \pi)$$

$$= k(\theta-\pi)/2\pi + 2k \ldots \quad (\pi \leq \theta < 2\pi)$$

Therefore, the distance $l_1(\theta)$ between $p_1(\theta)$ and $q_1(\theta)$ in the X' axis direction and the distance $l_2(\theta)$ between $p_2(\theta)$ and $q_2(\theta)$ in the X' axis direction are respectively expressed in accordance with the following expressions.

$$l_1(\theta) = q_1(\theta) - p_1(\theta) = -k\theta/\pi + 2k$$

$$l_2(\theta) = q_2(\theta) - p_2(\theta) = -k\theta/\pi + k \ldots \quad (0 \leq \theta < \pi)$$

$$= -k\theta/\pi + 3k \ldots \quad (\pi \leq \theta < 2\pi)$$

It should be noted that $l_1(\theta) > l_2(\theta)$ where $0 \leq \theta < \pi$ and $l_1(\theta) < l_2(\theta)$ where $\pi \leq \theta < 2\pi$.

As it will be understood from the description above, the distance $l_1(\theta)$ and the distance $l_2(\theta)$ which can be acquired from the marker portion 15a in the tomographic image are functions of the angle $\theta$ of the surgical tool 7 around the X' axis. When at least one of the distance $l_1(\theta)$ or the distance $l_2(\theta)$ is determined, single $\theta$ is determined.

As an example, for example, it is assumed that the distance $l_1(\theta)$ is k. In this case, $-k\theta/\pi + 2k = k$, and $\theta = \pi$ is established on the basis of this expression. In this manner, the angle $\theta$ around the X' axis in the resin portion 12 can be detected on the basis of at least one of the distance $l_1(\theta)$ or the distance $l_2(\theta)$. That is, the angle $\theta$ (attitude) around the X' axis in the surgical tool 7 can be detected on the basis of information regarding the marker portion 15a.

Further, when the angle $\theta$ of the surgical tool 7 around the X' axis is determined, the position of the distal end of the surgical tool 7 can be recovered. As an example, for example, it is assumed that $\theta$ is $\pi$. In this case, in the X' axis direction, the position of $p_1(\theta)$ is k/2 and the point-of-origin (the foremost end position of the first marker 16) exists between the position of $p_1(\theta)$ and the position of -k/2.

Here, provided that in the X' axis direction, a distance between a position of the point-of-origin and a position of the distal end of the surgical tool 7 is D, the distal end of the surgical tool 7 exists between the position of the point-of-origin to, in addition, the position of -D. In this manner, the position of the distal end end of the surgical tool 7 can also be detected on the basis of information regarding the marker portion 15a.

It should be noted that the first marker 16 and the second marker 17 only need to be arranged in the resin portion 12 such that when the surgical tool 7 is rotated around the X' axis, the distances $l_1(\theta)$ and $l_2(\theta)$ between the first marker 16 and the second marker 17 in the X' axis direction vary in accordance with this rotation in the tomographic image along the longitudinal direction (X' axis direction) of the surgical tool 7.

[Configuration (2) of marker Portion 15]

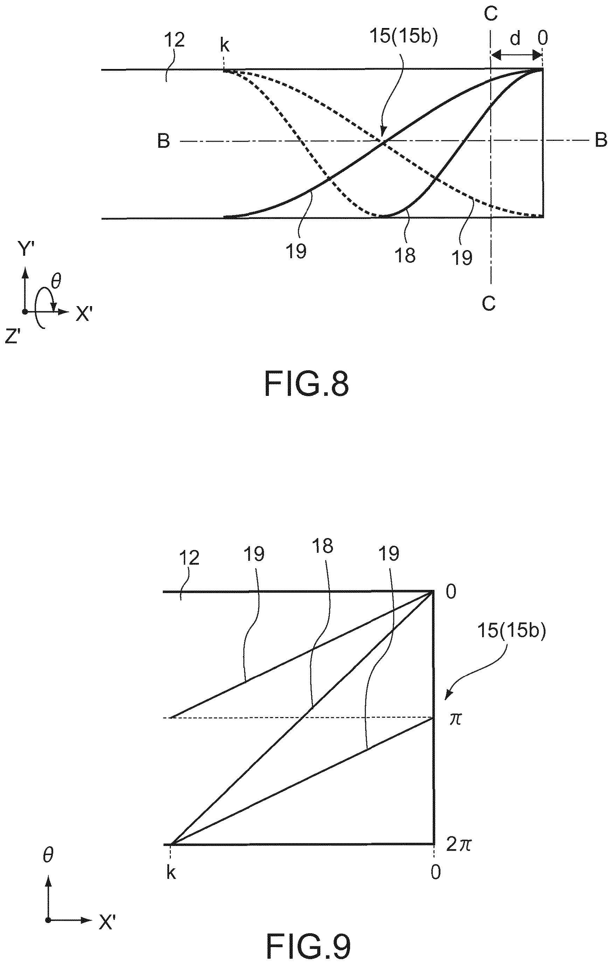

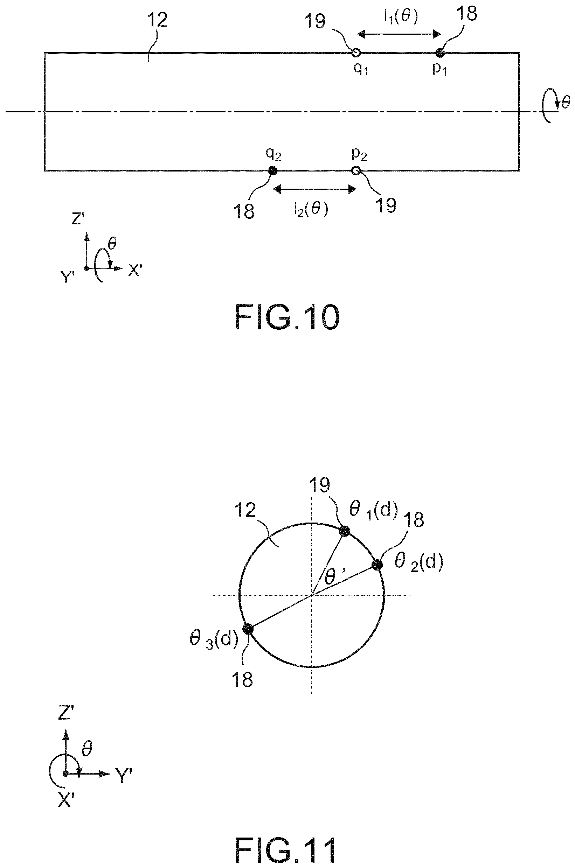

Next, another example of a configuration of the marker portion 15 will be described. Fig. 8 is an enlarged diagram showing a marker portion 15b. Fig. 9 is a net showing the marker portion 15b. It should be noted that in Figs. 8 and 9, the right-hand side is the distal-end side (the same applies to Fig. 10 to be described later). Further, in Fig. 9, the vertical direction is the $\theta$ direction (the direction around the X' axis) and the horizontal direction is the X' axis direction (the longitudinal direction).

As shown in those figures, the marker portion 15b includes a single first marker 18 and two second markers 19. The first marker 18 is wound once around the resin portion 12 in the particular direction in the $\theta$ direction in a spiral form. Further, the two second markers 19 are each half wound around the resin portion 12 in the same direction as the particular direction in a spiral form with the phase deviated by $\pi$.

It should be noted that the distance in the X' axis direction between the foremost end position and the rearmost end position of the first marker 18 and the distance in the X' axis direction between the foremost end position and the rearmost end position of the two second markers 19 are each equally set to k.

Fig. 10 is a diagram showing the resin portion 12 and the marker portion 15b which are shown in the tomographic image in a case where an X'Z' plane indicated as B-B in Fig. 8 is set as the tomographic surface.

In the tomographic image in Fig. 10, the first marker 18 wound around the resin portion 12 crosses at a total of two points, at a single point of each of the upper and lower sides of the resin portion 12 with respect to the X'Z' plane. It is assumed that the upper point at this time is $p_1(\theta)$ and the lower point is $q_2(\theta)$.

Similarly, the two second markers 19 wound around the resin portion 12 cross at a total of two points, at a single point of each of the upper and lower sides of the resin portion 12 with respect to the X'Z' plane. It is assumed that the upper point at this time is $q_1(\theta)$ and the lower point is $p_2(\theta)$.

It should be noted that in the X' axis direction, the foremost end position of the first marker 18 is set as the point-of-origin (i.e., 0) and a direction toward the back side from this point-of-origin is set as a positive direction. Further, the value of $\theta$ is set to be 0 when the foremost end position in the first marker 18 cross the X'Z' plane and the angle around the X' axis in the resin portion 12 is set to use the angle at this time as the criteria. Further, the clockwise direction is set as the positive direction in the $\theta$ direction as the resin portion 12 is viewed from the distal-end side.

At this time, the respective points of $p_1(\theta)$, $p_2(\theta)$, $q_1(\theta)$, and $q_2(\theta)$ are respectively expressed in accordance with the following expressions. Where the value of k is the distance in the X' axis direction between the foremost end position and the rearmost end position of the first marker 18 and is also the distance in the X' axis direction between the foremost end position and the rearmost end position of the two second markers 19.

$$p_1(\theta) = k\theta/2\pi$$

$$q_1(\theta) = k\theta/\pi \ldots \ldots \quad (0 \leq \theta < \pi)$$

$$= k(\theta-\pi)/\pi \ldots \quad (\pi \leq \theta < 2\pi)$$

$$p_2(\theta) = k\theta/\pi \ldots \ldots \quad (0 \leq \theta < \pi)$$

$$= k(\theta-\pi)/\pi \ldots \quad (\pi \leq \theta < 2\pi)$$

$$q_2(\theta) = k(\theta + \pi)/2\pi \quad (0 \leq \theta < \pi)$$

$$= k(\theta-\pi)/2\pi \quad (\pi \leq \theta < 2\pi)$$

Therefore, the distance $l_1(\theta)$ between $p_1(\theta)$ and $q_1(\theta)$ in the X' axis direction and the distance $l_2(\theta)$ in the X' axis direction between $p_2(\theta)$ and $q_2(\theta)$ are respectively expressed in accordance with the following expressions.

$$l_1(\theta) = q_1(\theta) - p_1(\theta) = k\theta/2\pi \ldots\ldots \quad (0 \leq \theta < \pi)$$

$$= p_1(\theta) - q_1(\theta) = -k\theta/2\pi + k \ldots \quad (\pi \leq \theta < 2\pi)$$

$$l_2(\theta) = q_2(\theta) - p_2(\theta) = -k\theta/2\pi + k/2. \quad (0 \leq \theta < \pi)$$

$$= p_2(\theta) - q_2(\theta) = k\theta/2\pi - k/2.. \quad (\pi \leq \theta < 2\pi)$$

As it will be understood from the description above, the distance $l_1(\theta)$ and the distance $l_2(\theta)$ which can be acquired from the marker portion 15b in the tomographic image are functions of the angle $\theta$ of the surgical tool 7 around the X' axis. Then, when at least one of the distance $l_1(\theta)$ or the distance $l_2(\theta)$ is determined, single $\theta$ is determined or two candidates as $\theta$ are determined.

As an example, for example, it is assumed that the distance $l_1(\theta)$ is k/2. In this case, $k\theta/2\pi$ = k/2 or $-k\theta/2\pi$ + k = k/2 is established and $\theta = \pi$ is established in accordance with those expressions, such that single $\theta$ is determined (it should be noted that the former expression has a condition of $(0 \leq \theta < \pi)$, and thus the later expression is adopted).

Typically, single $\theta$ is not determined and two candidates are determined in this manner. As an example, for example, it is assumed that the distance $l_1(\theta)$ is k/4. In this case, $k\theta/2\pi$ = k/4 or $-k\theta/2\pi$ + k = k/4 and $\theta = \pi/2$ or $\theta = 3\pi/2$ is established in accordance with those expressions, such that two candidates are determined as $\theta$.

In this case, a smaller value $s_1$ of the two points $p_1(\theta)$ and $q_1(\theta)$ on the upper side of the resin portion 12 is compared with a smaller value $s_2$ of the two points $p_2(\theta)$ and $q_2(\theta)$ on the lower side. Then, $0 \leq \theta < \pi$ where $s_1 \leq s_2$, and $\pi \leq \theta < 2\pi$ where $s_1 > s_2$.

In this manner, the angle $\theta$ around the X' axis in the resin portion 12 can be detected on the basis of values including at least one value of the distance $l_1(\theta)$ or the distance $l_2(\theta)$, $s_1$, $s_2$, and the like. That is, the angle $\theta$ (attitude) around the X' axis in the surgical tool 7 can be detected on the basis of information regarding the marker portion 15b.

Further, when the angle $\theta$ of the surgical tool 7 around the X' axis is determined, the position of the distal end of the surgical tool 7 can be recovered. As an example, for example, it is assumed that $\theta$ is $\pi$. In this case, in the X' axis direction, the position of $p_1(\theta)$ is k/2 and the point-of-origin (the foremost end position of the first marker 18) exists between the position of $p_1(\theta)$ and the position of -k/2.

Here, provided that in the X' axis direction, a distance between the position of the point-of-origin and the position of the distal end of the surgical tool 7 is D, the distal end of the surgical tool 7 exists between the position of the point-of-origin and, in addition, the position of -D. In this manner, the position of the distal end of the surgical tool 7 can also be detected on the basis of information regarding the marker portion 15b.

It should be noted that also in this example, the first marker 18 and the second markers 19 are arranged in the resin portion 12 such that when the surgical tool 7 is rotated around the X' axis in the tomographic image along the longitudinal direction (X' axis direction) of the surgical tool 7, the distances $l_1(\theta)$ and $l_2(\theta)$ between the first marker 18 and the second markers 19 in the X' axis direction vary in accordance with this rotation.

Fig. 11 is a diagram showing the resin portion 12 and the marker portion 15b which are shown in the tomographic image in a case where the Y'Z' plane indicated as C-C in Fig. 8 is set as the tomographic surface.

In the tomographic image in Fig. 11, the first marker 18 wound around the resin portion 12 crosses the Y'Z' plane at a single point. The point at this time is set as $\theta_1(d)$. Similarly, the two second markers 19 wound around the resin portion 12 each cross the Y'Z' plane at a single point. The points at this time are respectively set as $\theta_2(d)$ and $\theta_3(d)$.

At this time, $\theta_2(d)$ and $\theta_3(d)$ according to the two second markers 19 constantly face each other even if the tomographic surface is moved in the X' axis direction. The positions of the respective points are $\theta_1(d)$ = 2$\pi$d/k, $\theta_2(d)$ = $\pi$d/k, and $\theta_3(d)$ = $\pi$d/k + $\pi$. It should be noted that d is a distance between a point-of-origin 0 and the tomographic surface in the X' axis direction.

Here, $\theta_2(d)$ and $\theta_3(d)$ according to the two second markers 19 are linked with a straight line and an angle between this straight line and $\theta_1(d)$ according to the first marker 18 is set to be $\theta'(d)$ (0 < $\theta'$ < $\pi/2$). In this case, $\theta'(d)$ = $\theta_1(d)$ - $\theta_2(d)$ or $\theta'(d)$ = $\theta_3(d)$ - $\theta_1(d)$ is established. In this case, $\theta'(d)$ = $\pi$d/k is established.

As it will be understood from the description above, $\theta'(d)$ which can be acquired from the marker portion 15b in the tomographic image is a function of a distance d in the longitudinal direction (X' axis direction) of the surgical tool 7. Therefore, the position of the point-of-origin in the X' axis direction can be detected on the basis of this distance d and the position of the distal end of the surgical tool 7 can be detected in a manner similar to that described above.

It should be noted that the first marker 18 and the second markers 19 only need to be arranged in the resin portion 12 such that when the surgical tool 7 is rotated around the X' axis in the tomographic image along a direction orthogonal to the length direction of the surgical tool 7, the angle between the first marker 18 and the second markers 19 around the X' axis varies in accordance with this rotation.

It should be noted that even in the case where the tomographic image is the tomographic image in the direction along the longitudinal direction of the surgical tool 7 or even in the case where it is the tomographic image in the direction orthogonal to this direction, the first marker 18 and the second markers 19 according to the example here can suitably cope with it.

<Operation Description>

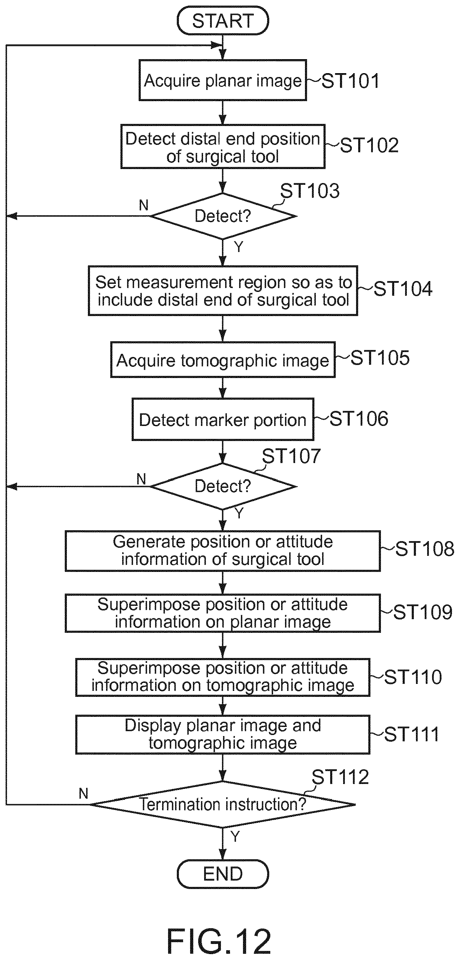

Next, processing of the control unit 1 in the surgical microscope apparatus 10 will be described. Fig. 12 is a flowchart showing the processing of the control unit 1. As shown in Fig. 12, first of all, the control unit 1 controls the front image acquisition unit 2, captures a front image of the eye, and acquires the captured front image of the eye from the front image acquisition unit 2 (Step 101).

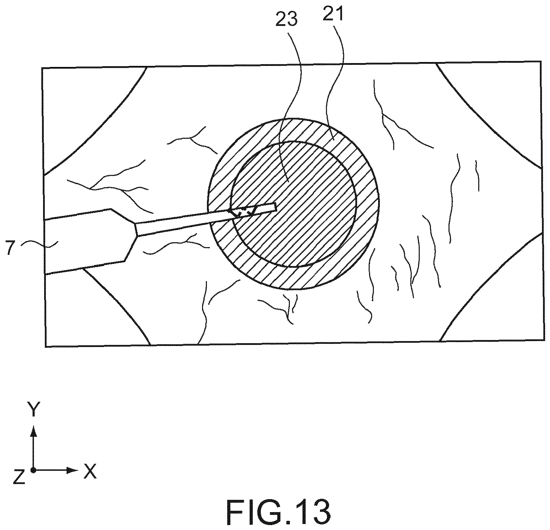

Fig. 13 is a diagram showing an example of the front image acquired by the front image acquisition unit 2. In the front image shown in Fig. 13, a state when the interior of the lens 23 is absorbed and removed through the surgical tool 7 for absorption in cataract surgery is shown.

When acquiring the front image, the control unit 1 executes image recognition in the front image and detects a position of the distal end of the surgical tool 7 (Step 102). It should be noted that the position and attitude of the surgical tool 7 is correctly detected by detecting the marker portion 15 after that, and thus detection of the position of the distal end of the surgical tool 7 in this step is executed as detection of a rough position.

If the control unit 1 cannot detect a position of the distal end of the surgical tool 7 in image recognition (NO in Step 103), the control unit 1 returns to Step 101 and acquires a front image again.

On the other hand, if the control unit 1 can detect the position of the distal end of the surgical tool 7 (YES in Step 103), the control unit 1 sets a measurement region (linear or planar) for executing scan in the tomographic image so as to include the distal end of the surgical tool 7 (Step 104).

When the control unit 1 sets the measurement region, the control unit 1 executes scan in the measurement region and acquires a tomographic image through the tomographic image acquisition unit 3 (Step 105) .

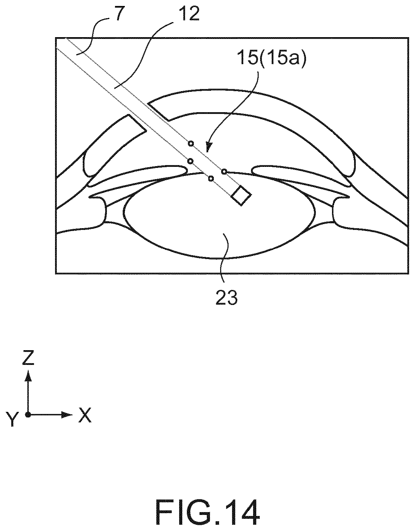

Fig. 14 is a diagram showing an example of the tomographic image acquired by the tomographic image acquisition unit 3. In the tomographic image shown in Fig. 14, a state when the interior of the lens 23 is absorbed and removed by the surgical tool 7 for absorption in the cataract surgery is shown.

As shown in Fig. 14, in this embodiment, the side of the distal end of the surgical tool 7 is constituted by the resin portion 12, and thus generation of shadows on the lower side of the surgical tool 7 and generation of artifacts around the surgical tool 7 are prevented. On the other hand, the resin portion 12 is displayed darker in the tomographic image and it is difficult to recognize the position of the surgical tool 7. However, the marker portion 15 provided in the resin portion 12 is detected as a high signal.

When the control unit 1 acquires the tomographic image, the control unit 1 executes detection processing of the marker portion 15 on the basis of the tomographic image (Step 106). In the detection processing of the marker portion 15, the control unit 1 detects a position or attitude of the surgical tool 7 in accordance with the above-mentioned method on the basis of information regarding the detected marker portion 15 (see [Configuration (1) of marker Portion 15], [Configuration (2) of marker Portion 15]). In this manner, by detecting the position or attitude of the surgical tool 7 on the basis of the marker portion 15, the position or attitude of the surgical tool 7 in the tomographic image can be correctly detected.

If the control unit 1 can detect the marker portion 15 (NO in Step 107), the control unit 1 returns to Step 101 and acquires a front image again. On the other hand, if the control unit 1 can detect the marker portion 15 (YES in Step 107), the control unit 1 generates position or attitude information of the surgical tool 7 for presenting the position or attitude of the surgical tool 7 to the user on the basis of the detected position or attitude of the surgical tool 7 (Step 108).

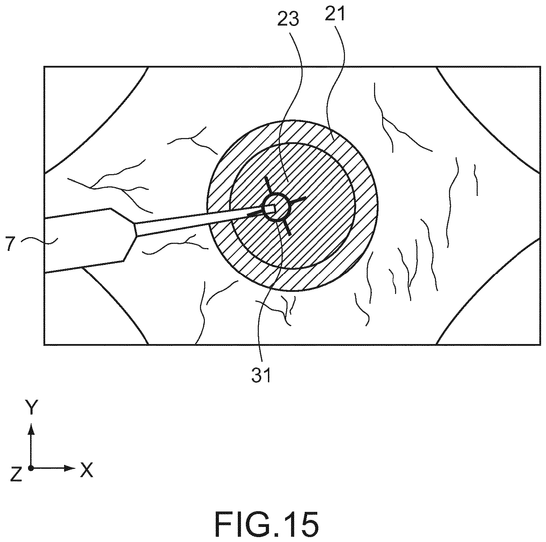

Next, the control unit 1 superimposes the generated position or attitude information of the surgical tool 7 on the front image (Step 109). Fig. 15 is a diagram showing a state when the position or attitude information of the surgical tool 7 is superimposed on the front image. In the example shown in Fig. 15, a state when a mark 31 indicating the position of the distal end of the surgical tool 7 is arranged as the position or attitude information in the position of the distal end of the surgical tool 7 is shown.

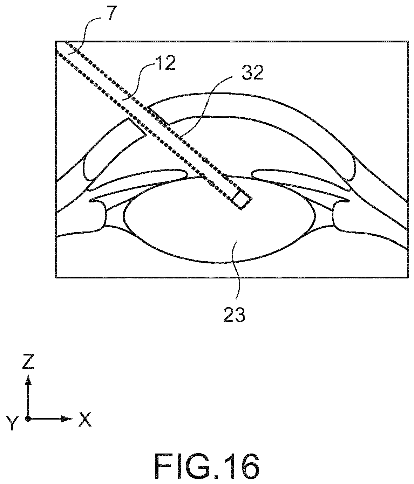

Next, the control unit 1 superimposes the generated position or attitude information of the surgical tool 7 on the tomographic image (Step 110). Fig. 16 is a diagram showing a state when the position or attitude information of the surgical tool 7 is superimposed on the tomographic image. In the example shown in Fig. 16, a state when a mark 32 indicating the outline of the surgical tool 7 in a position corresponding to the outline of the surgical tool 7 is arranged as the position or attitude information is shown.

When the control unit 1 superimposes the position or attitude information of the surgical tool 7 on the tomographic image, then the control unit 1 causes the display unit 5 to display the front image and the tomographic image on which the position or attitude information of the surgical tool 7 is superimposed (Step 111). It should be noted that the front image and the tomographic image may be displayed on the same display unit 5 or may be displayed on discrete display units 5.

Next, the control unit 1 determines whether or not the termination instruction is input by the user (Step 112). If the termination instruction is not input (NO in Step 112), the control unit 1 returns to Step 101. Otherwise, if the termination instruction is input (YES in Step 112), the processing is terminated.

<Actions, etc.>

As described hereinabove, in this embodiment, the control unit 1 acquires information regarding the marker portion 15 shown in a tomographic image of an eye which is subjected to a surgical operation through the surgical tool 7 including the resin portion 12 including the marker portion 15 and detects a position or attitude of the surgical tool 7 on the basis of the information regarding the marker portion 15. With this configuration, even in a case where the surgical tool 7 is constituted by a resin, the control unit 1 can correctly detect the position or attitude of the surgical tool 7 in the tomographic image.

Further, in this embodiment, the position or attitude information of the surgical tool 7 for presenting the position or attitude of the surgical tool 7 to the user is generated on the basis of the detected position or attitude of the surgical tool 7, and this position or attitude information is arranged in the front image and the tomographic image. The user can perform a surgical operation for the eye while visually recognizing the position or attitude information, and thus generation of the surgical errors can be prevented.

In particular, in this embodiment, even if the resin portion 12 is displayed darker in the tomographic image and it is difficult to recognize the position of the surgical tool 7, the user can clearly recognize the position or attitude of the surgical tool 7 by visually recognizing the position or attitude information of the surgical tool 7.

Further, as described above, in this embodiment, the position or attitude of the surgical tool 7 can be correctly detected, and thus the position or attitude information of the surgical tool 7 for presenting to the user can also be correctly generated. Therefore, the user can correctly recognize the position or attitude of the surgical tool 7.

Further, in this embodiment, as described above, the marker portion 15 is encoded as a suitable-shape geometric pattern, and thus detection of the position or attitude of the surgical tool 7 and the position or attitude information of the surgical tool 7 to be presented to the user becomes more correct.

<<Second Embodiment>>

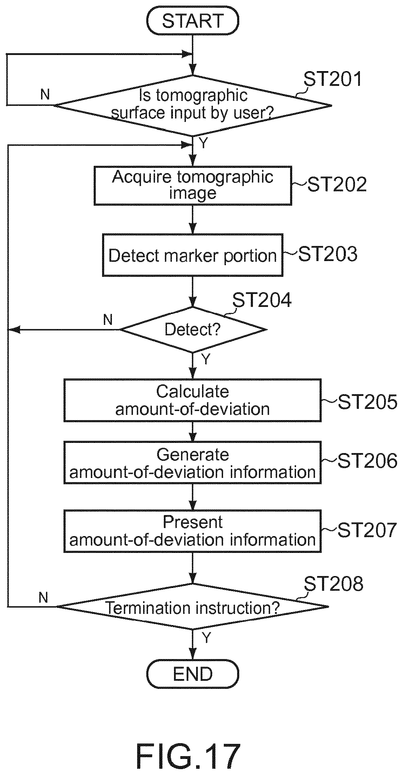

Next, a second embodiment of the present technology will be described. Fig. 17 is a flowchart showing processing according to the second embodiment.

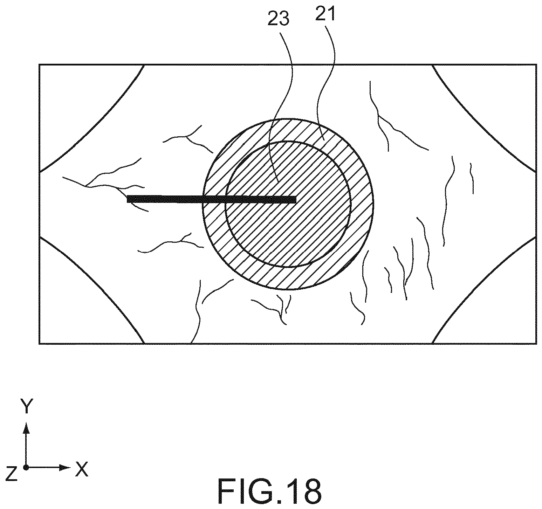

First of all, the control unit 1 determines whether or not the tomographic surface is input by the user (Step 201). Fig. 18 is a diagram showing an example when the tomographic surface is input by the user. As shown in Fig. 18, for example, the user inputs a tomographic surface (see thick line) via the input unit 6 while visually recognizing the front image of the eye displayed on the display unit 5. This tomographic surface is set as a target for positioning the surgical tool 7.

In a case where the tomographic surface is input by the user (YES in Step 201), the control unit 1 acquires the tomographic image through the tomographic image acquisition unit 3 in accordance with the input tomographic surface (Step 202). Next, the control unit 1 executes detection processing of the marker portion 15 on the basis of the acquired tomographic image (Step 203) .

If the marker portion 15 is not detected (NO in Step 204), the control unit 1 returns to Step 202 and acquires the tomographic image. On the other hand, if the marker portion 15 is detected (YES in Step 204), the control unit 1 calculates an amount-of-deviation with respect to a tomographic surface of the surgical tool 7 on the basis of information regarding the detected marker portion 15 (on the basis of the position or attitude of the surgical tool 7).

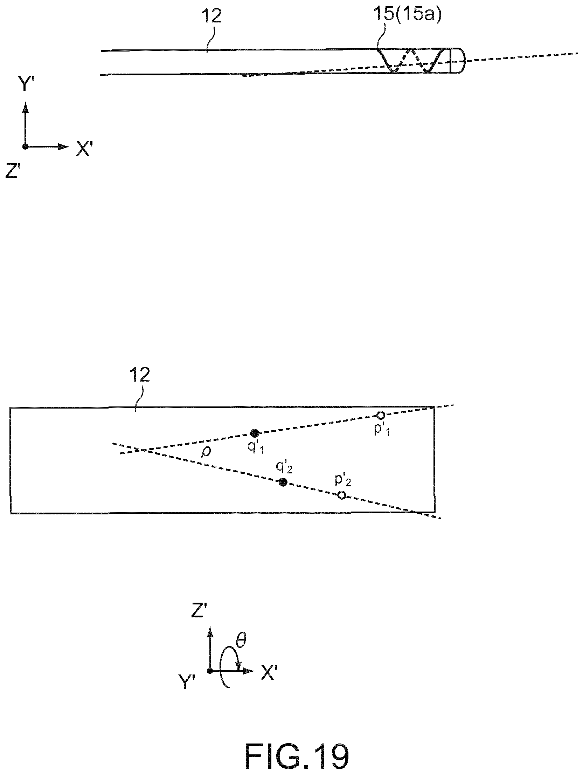

Figs. 19 to 21 each are a diagram showing a state in which the surgical tool 7 is deviated with respect to the tomographic surface. In each of Figs. 19 to 21, the attitude of the surgical tool 7 with respect to the tomographic surface is shown on an upper side and the position of the marker portion 15 in a case where the tomographic image is acquired with respect to the tomographic surface in the upper representation is shown on a lower side.

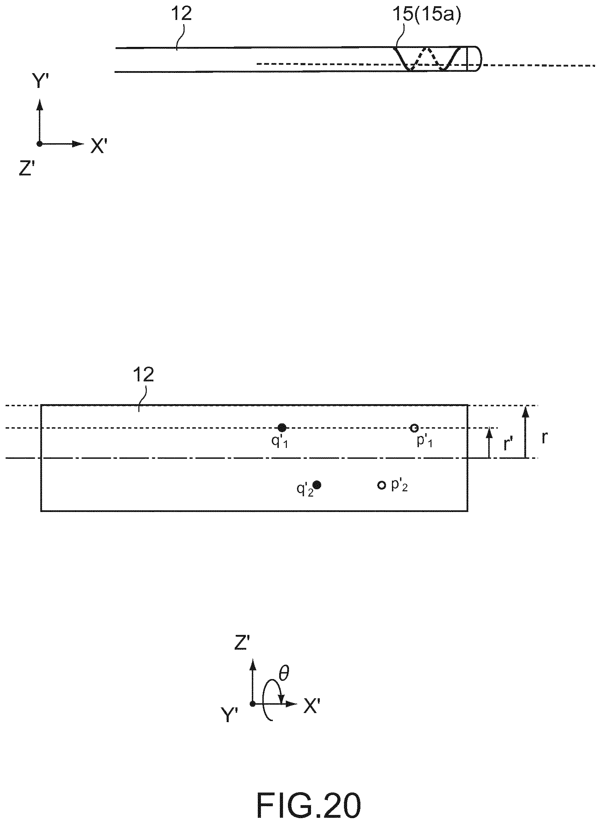

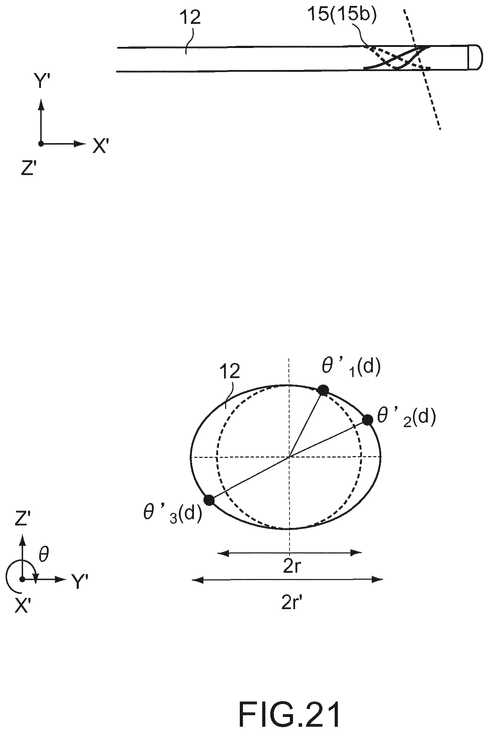

It should be noted that in Figs. 19 and 20, an example in a case where the marker portion 15a is used is shown and in Fig. 21, a state when the marker portion 15b is used is shown.

Referring to the upper side of Fig. 19, in this example, the longitudinal direction (X' axis direction) of the surgical tool 7 is not parallel to the direction of the tomographic surface and is deviated. In this case, as shown on the lower side of Fig. 19, positions of the four points $p_1'(\theta)$, $p_2'(\theta)$, $q_1'(\theta)$, and $q_2'(\theta)$ according to the marker portion 15 in the tomographic image are deviated in comparison with original positions of the four points.

That is, these four points should be originally positioned at two points in positions of each of the upper and lower ends of the resin portion 12 in parallel. However, these four points are not located at the upper and lower ends of the resin portion 12 and are not parallel. In this case, the control unit 1 calculates an amount-of-deviation $\varepsilon$ in accordance with Expression $\varepsilon = \cos(\rho)$. It should be noted that $\rho$ is an angle formed by a straight line linking the two upper points and a straight line linking the two lower points.

Referring to the upper side of Fig. 20, in this example, the longitudinal direction (X' axis direction) of the surgical tool 7 is parallel to the direction of the tomographic surface. However, the position in the Y' axis direction in the surgical tool 7 is deviated with respect to the tomographic surface.

In this case, as shown on the lower side of Fig. 20, the positions of the four points $p_1'(\theta)$, $p_2'(\theta)$, $q_1'(\theta)$, and $q_2'(\theta)$ according to the marker portion 15 in the tomographic image are deviated in comparison with the original positions of the four points. That is, these four points should be originally positioned at the upper and lower ends of the resin portion 12. However, the upper and lower ends of the resin portion 12 are not located (but those are parallel).

In this case, the control unit 1 calculates an amount-of-deviation $\varepsilon$ in accordance with Expression $\varepsilon = r - r'$. It should be noted that r is a radius of the resin portion 12 and r' is a distance between the center axis and a detected point.

Referring to the upper side of Fig. 21, in this example, the longitudinal direction (X' axis direction) of the surgical tool 7 is not perpendicular to the direction of the tomographic surface and is deviated.

In this case, as shown on the lower side of Fig. 21, positions of three points $\theta_1'(d)$, $\theta_2'(d)$, and $\theta_3'(d)$ according to the marker portion 15 in the tomographic image are deviated in comparison with original positions of three points. That is, a circular shape should be originally obtained when linking this three points. However, an elliptical shape is obtained.

In this case, the control unit 1 calculates an amount-of-deviation $\varepsilon$ in accordance with Expression $\varepsilon = 2(r'-r)$. It should be noted that 2r is a radius of the resin portion 12 and r' is a major axis of the ellipse estimated on the basis of the three points.

Referring back to Fig. 17, when the control unit 1 calculates an amount-of-deviation, then the control unit 1 generates amount-of-deviation information for presenting the amount-of-deviation to the user (Step 206). Then, the control unit 1 presents the generated amount-of-deviation information to the user (Step 207) and terminates processing if a termination instruction is provided (YES in Step 208),.

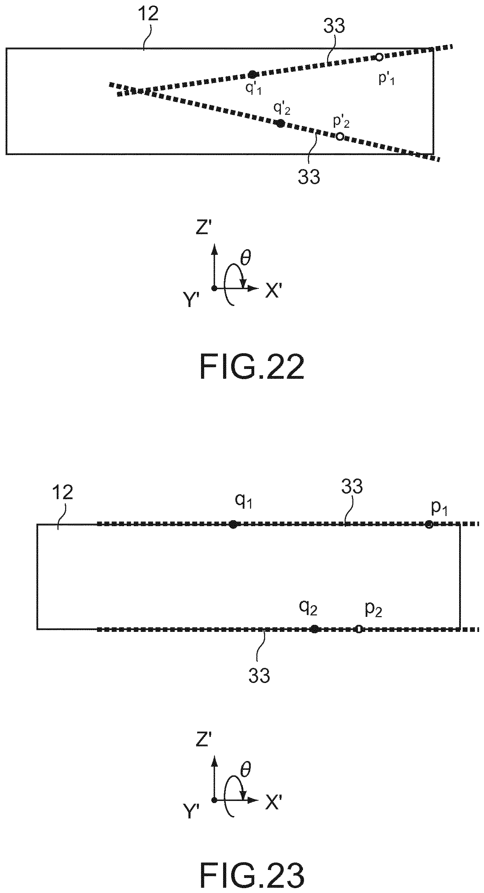

Figs. 22 and 23 are diagrams each showing an example of amount-of-deviation information presented to the user. As shown in Figs. 22 and 23, the information regarding the amount-of-deviation is arranged in the tomographic image. This amount-of-deviation information is generated on the basis of the position of the marker portion 15 in the tomographic image and is constituted by two straight lines including a straight line 33 linking the two upper points of the resin portion 12 and the straight line 33 linking the two lower points.

Fig. 22 shows an example in a case where the two straight lines 33 are not parallel and positioning of the surgical tool 7 is not suitable. On the other hand, in Fig. 23, an example in a case where the two straight lines 33 are parallel and positioning of the surgical tool 7 is suitable is shown.

It should be noted that the amount-of-deviation information is not limited to the examples shown in Figs. 22 and 23. For example, the amount-of-deviation information may be a graph, an indicator, and the like. Any type of amount-of-deviation information can be employed as long as the user can recognize the amount-of-deviation. Further, the amount-of-deviation information is not limited to the tomographic image and may be arranged in the front image.

In the second embodiment, the amount-of-deviation of the position or attitude of the surgical tool 7 is detected with respect to the tomographic surface for acquiring the tomographic image and the amount-of-deviation information for presenting the amount-of-deviation to the user is generated on the basis of the detected amount-of-deviation. Therefore, by referring to the presented amount-of-deviation, the user can position the surgical tool 7 in a correct position.

Further, the amount-of-deviation can detected through the marker portion 15, and thus it is possible to correctly detect the amount-of-deviation of the position or attitude of the surgical tool 7 with respect to the tomographic surface and to present correct deviation information to the user. Further, as shown in Figs. 22 and 23, suitable amount-of-deviation information can be generated by generating an amount-of-deviation on the basis of the position of the marker portion 15 for presenting to the user.

In the description of the second embodiment, the case where the user adjusts the position of the surgical tool 7 by using the tomographic surface as a reference has been described. On the other hand, the position of the tomographic surface may be determined by using the surgical tool 7 as a reference.

In this case, first of all, the control unit 1 performs image recognition on the front image to thereby acquire a rough position of the distal end of the surgical tool 7, sets a tomographic surface by using the distal end of this surgical tool 7 as a reference, and acquires a tomographic image. Then, an accurate position of the distal end in the surgical tool 7 is detected on the basis of the information regarding the marker portion 15 in the obtained tomographic image.

Then, the control unit 1 calculates the amount-of-deviation between the tomographic surface and the position of the distal end of the surgical tool 7 on the basis of the correctly detected position of the distal end of the surgical tool 7, and changes the tomographic surface such that the amount-of-deviation becomes smaller on the basis of the amount-of-deviation. By such processing, the tomographic surface can be set at a suitable position in accordance with the position or attitude of the surgical tool 7.

<<Various Modified Examples>>

In the description above, the case where the marker portion 15 is provided in the surface of the resin portion 12 has been described. However, the marker portion 15 may be arranged inside the resin portion 12.

In the description above, the case where the control unit 1 in the surgical microscope apparatus 10 executes the above-mentioned various types of processing has been described. On the other hand, the above-mentioned various types of processing may be executed by the control unit 1 of the server apparatus over the network (information processing apparatus).

Reference Signs List

| | |
|---|---|
| 1 | control unit |
| 2 | front image acquisition unit |
| 3 | tomographic image acquisition unit |
| 7 | surgical tool |
| 10 | surgical microscope apparatus |
| 12 | resin portion |
| 15 | marker portion |
| 16, 18 | first marker |
| 17, 19 | second marker |

**Claims**

1. An information processing apparatus, comprising:

   a control unit (1) configured to acquire information regarding a marker portion (15) shown in a tomographic image of an eye which is subjected to a surgical operation through a surgical tool (7) including a resin portion (12) including the marker portion; wherein
   the marker portion includes a particular geometric pattern; and wherein
   the marker portion includes a first marker (16) and a second marker (17), and
   the first marker and the second marker are arranged in the resin portion such that when the surgical tool is rotated around an axis along a longitudinal direction of the surgical tool, a distance between the first marker and the second marker in the longitudinal direction varies in accordance with the rotation in a tomographic image along the longitudinal direction and/or an angle between the first marker and the second marker around the axis varies in accordance with the rotation in the tomographic image along a direction orthogonal to the length direction; and wherein the control unit is configured to detect a position or rotation angle ($\Theta$) around the axis (X) of the surgical tool on a basis of the information regarding the marker portion.

2. The information processing apparatus according to claim 1, wherein
   the control unit is configured to generate, on a basis of the detected position or rotation angle of the surgical tool, position or rotation angle information of the surgical tool for presenting the position or rotation angle of the surgical tool to a user.

3. The information processing apparatus according to claim 2, wherein
   the control unit is configured to arrange the position or rotation angle information of the surgical tool in the tomographic image.

4. The information processing apparatus according to claim 2, wherein
   the control unit is configured to arrange the position or rotation angle information of the surgical tool in a front image of the eye.

**5.** The information processing apparatus according to claim 1, wherein
the control unit is configured to detect an amount-of-deviation of the position or rotation angle of the surgical tool with respect to a tomographic surface for acquiring the tomographic image on a basis of the detected position or rotation angle of the surgical tool.

**6.** The information processing apparatus according to claim 5, wherein
the control unit is configured to generate amount-of-deviation information for presenting the amount-of-deviation to a user on a basis of the detected amount-of-deviation.

**7.** The information processing apparatus according to claim 6, wherein
the control unit is configured to generate the amount-of-deviation information on a basis of the information regarding the marker portion.

**8.** The information processing apparatus according to claim 5, wherein
the control unit is configured to change the tomographic surface on a basis of the detected amount-of-deviation.

**9.** A surgical tool (7), comprising

a resin portion (12) including a marker portion (15) provided for detecting a position or rotation angle ($\Theta$) around an axis (X) along a longitudinal direction of a surgical tool in a tomographic image of an eye which is subjected to a surgical operation through the surgical tool; wherein
the marker portion includes a particular geometric pattern; in which
the marker portion includes a first marker (16) and a second marker (17), and
the first marker and the second marker are arranged in the resin portion such that when the surgical tool is rotated around the axis along a longitudinal direction of the surgical tool, a distance between the first marker and the second marker in the longitudinal direction varies in accordance with the rotation in the tomographic image along the longitudinal direction and/or an angle between the first marker and the second marker around the axis varies in accordance with the rotation in the tomographic image along a direction orthogonal to the length direction.

**10.** An information processing method, comprising:

acquiring, by a control unit (1), information regarding a marker portion (15) shown in a tomographic image of an eye which is subjected to a surgical operation through a surgical tool (7) including a resin portion (12) including the marker portion; and wherein
the marker portion includes a particular geometric pattern; and wherein
the marker portion includes a first marker (16) and a second marker (17), and
the first marker and the second marker are arranged in the resin portion such that when the surgical tool is rotated around an axis along a longitudinal direction of the surgical tool, a distance between the first marker and the second marker in the longitudinal direction varies in accordance with the rotation in a tomographic image along the longitudinal direction and/or an angle between the first marker and the second marker around the axis varies in accordance with the rotation in the tomographic image along a direction orthogonal to the length direction;
detecting, by the control unit, a position or rotation angle ($\Theta$) around the axis (X), of the surgical tool on a basis of the information regarding the marker portion.

**11.** A program that causes a computer to execute:
a method according to Claim 10.

## Patentansprüche

**1.** Informationsverarbeitungsvorrichtung, umfassend: eine Steuereinheit (1), die ausgestaltet ist zum Erlangen von Informationen zu einem Markerabschnitt (15), der in einem tomographischen Bild eines Auges gezeigt wird, das einem chirurgischen Eingriff durch ein chirurgisches Werkzeug (7) unterzogen wird, das einen Harzabschnitt (12) einschließt, der den Markerabschnitt einschließt; wobei

der Markerabschnitt ein spezielles geometrisches Muster einschließt; und wobei

der Markerabschnitt einen ersten Marker (16) und einen zweiten Marker (17) einschließt, und
der erste Marker und der zweite Marker in dem Harzabschnitt so angeordnet sind, dass, wenn das chirurgische Werkzeug um eine Achse entlang einer Längsrichtung des chirurgischen Werkzeugs rotiert wird, ein Abstand zwischen dem ersten Marker und dem zweiten Marker in der Längsrichtung gemäß der Rotation in einem tomographischen Bild entlang der Längsrichtung variiert und/oder ein Winkel zwischen dem ersten Marker und dem zweiten Marker um die Achse gemäß der Rotation in dem tomographischen Bild entlang einer Richtung orthogonal zu der Längsrichtung variiert; und
wobei die Steuereinheit ausgestaltet ist, um eine Position oder einen Rotationswinkel ($\theta$) um die Achse (X) des chirurgischen Werkzeugs basierend auf den Informationen zu dem Markerabschnitt zu detektieren.

2. Informationsverarbeitungsvorrichtung nach Anspruch 1, wobei
die Steuereinheit ausgestaltet ist, um basierend auf der detektierten Position oder dem detektierten Rotationswinkel des chirurgischen Werkzeugs Positions- oder Rotationswinkelinformationen des chirurgischen Werkzeugs zu generieren, um die Position oder den Rotationswinkel des chirurgischen Werkzeugs einem Anwender zu präsentieren.

3. Informationsverarbeitungsvorrichtung nach Anspruch 2, wobei
die Steuereinheit ausgestaltet ist, um die Positions- oder Rotationswinkelinformationen des chirurgischen Werkzeugs in dem tomographischen Bild anzuordnen.

4. Informationsverarbeitungsvorrichtung nach Anspruch 2, wobei
die Steuereinheit ausgestaltet ist, um die Positions- oder Rotationswinkelinformationen des chirurgischen Werkzeugs in einem Frontbild des Auges anzuordnen.

5. Informationsverarbeitungsvorrichtung nach Anspruch 1, wobei
die Steuereinheit ausgestaltet ist, um einen Betrag der Abweichung von der Position oder dem Rotationswinkel des chirurgischen Werkzeugs in Bezug zu einer tomographischen Oberfläche zum Erlangen des tomographischen Bildes basierend auf der detektierten Position oder dem detektierten Rotationswinkel des chirurgischen Werkzeugs zu detektieren.

6. Informationsverarbeitungsvorrichtung nach Anspruch 5, wobei
die Steuereinheit ausgestaltet ist, um Informationen zum Betrag der Abweichung zu generieren, um den Betrag der Abweichung einem Anwender basierend auf dem detektierten Betrag der Abweichung zu präsentieren.

7. Informationsverarbeitungsvorrichtung nach Anspruch 6, wobei
die Steuereinheit ausgestaltet ist, um die Informationen zum Betrag der Abweichung basierend auf den Informationen zu dem Markerabschnitt zu generieren.

8. Informationsverarbeitungsvorrichtung nach Anspruch 5, wobei
die Steuereinheit ausgestaltet ist, um die tomographische Oberfläche basierend auf dem detektierten Betrag der Abweichung zu ändern.

9. Chirurgisches Werkzeug (7), umfassend:

einen Harzabschnitt (12), der einen Markerabschnitt (15) einschließt, der zum Detektieren einer Position oder eines Rotationswinkels ($\theta$) um eine Achse (X) entlang einer Längsrichtung eines chirurgischen Werkzeugs in einem tomographischen Bild eines Auges, das einem chirurgischen Eingriff durch das chirurgische Werkzeug unterzogen wird, bereitgestellt wird; wobei
der Markerabschnitt ein spezielles geometrisches Muster einschließt; bei dem
der Markerabschnitt einen ersten Marker (16) und einen zweiten Marker (17) einschließt, und
der erste Marker und der zweite Marker in dem Harzabschnitt so angeordnet sind, dass, wenn das chirurgische Werkzeug um die Achse entlang einer Längsrichtung des chirurgischen Werkzeugs rotiert wird, ein Abstand zwischen dem ersten Marker und dem zweiten Marker in der Längsrichtung gemäß der Rotation in dem tomographischen Bild entlang der Längsrichtung variiert und/oder ein Winkel zwischen dem ersten Marker und dem zweiten Marker um die Achse gemäß der Rotation in dem tomographischen Bild entlang einer Richtung orthogonal zu der Längsrichtung variiert.

10. Informationsverarbeitungsverfahren, umfassend:

Erlangen von Informationen zu einem Markerabschnitt (15), der in einem tomographischen Bild eines Auges gezeigt wird, das einem chirurgischen Eingriff durch ein chirurgisches Werkzeug (7) unterzogen wird, das einen Harzabschnitt (12) einschließt, der den Markerabschnitt einschließt, durch eine Steuereinheit (1); und wobei der Markerabschnitt ein spezielles geometrisches Muster einschließt; und wobei der Markerabschnitt einen ersten Marker (16) und einen zweiten Marker (17) einschließt, und der erste Marker und der zweite Marker in dem Harzabschnitt so angeordnet sind, dass, wenn das chirurgische Werkzeug um eine Achse entlang einer Längsrichtung des chirurgischen Werkzeugs rotiert wird, ein Abstand zwischen dem ersten Marker und dem zweiten Marker in der Längsrichtung gemäß der Rotation in einem tomographischen Bild entlang der Längsrichtung variiert und/oder ein Winkel zwischen dem ersten Marker und dem zweiten Marker um die Achse gemäß der Rotation in dem tomographischen Bild entlang einer Richtung orthogonal zu der Längsrichtung variiert;

Detektieren einer Position oder eines Rotationswinkels ($\theta$) um die Achse (X) des chirurgischen Werkzeugs basierend auf den Informationen zu dem Markerabschnitt durch die Steuereinheit.

**11.** Programm, welches bewirkt, dass ein Computer ein Verfahren gemäß Anspruch 10 ausführt.

## Revendications

**1.** Appareil de traitement d'informations, comprenant :

une unité de commande (1) configurée pour acquérir des informations concernant une partie marqueur (15) représentée dans une image tomographique d'un œil qui est soumis à une opération chirurgicale au moyen d'un outil chirurgical (7) comprenant une partie de résine (12) comprenant la partie marqueur ;

la partie marqueur comprenant un motif géométrique particulier ; et

la partie marqueur comprenant un premier marqueur (16) et un second marqueur (17), et

le premier marqueur et le second marqueur étant agencés dans la partie de résine de sorte que lorsque l'outil chirurgical est tourné autour d'un axe le long d'une direction longitudinale de l'outil chirurgical, une distance entre le premier marqueur et le second marqueur dans la direction longitudinale varie en fonction de la rotation dans une image tomographique le long de la direction longitudinale, et/ou un angle entre le premier marqueur et le second marqueur autour de l'axe varie en fonction de la rotation dans l'image tomographique le long d'une direction orthogonale à la direction longitudinale ; et

l'unité de commande étant configurée pour détecter une position ou un angle de rotation ($\theta$) autour de l'axe (X) de l'outil chirurgical sur la base des informations concernant la partie marqueur.

**2.** Appareil de traitement d'informations selon la revendication 1, l'unité de commande étant configurée pour générer, sur la base de la position ou de l'angle de rotation détecté de l'outil chirurgical, des informations de position ou d'angle de rotation de l'outil chirurgical pour présenter la position ou l'angle de rotation de l'outil chirurgical à un utilisateur.

**3.** Appareil de traitement d'informations selon la revendication 2, l'unité de commande étant configurée pour agencer les informations de position ou d'angle de rotation de l'outil chirurgical dans l'image tomographique.

**4.** Appareil de traitement d'informations selon la revendication 2, l'unité de commande étant configurée pour agencer les informations de position ou d'angle de rotation de l'outil chirurgical dans une image frontale de l'œil.

**5.** Appareil de traitement d'informations selon la revendication 1, l'unité de commande étant configurée pour détecter une quantité de déviation de la position ou de l'angle de rotation de l'outil chirurgical par rapport à une surface tomographique pour acquérir l'image tomographique sur une base de la position ou de l'angle de rotation détecté de l'outil chirurgical.

**6.** Appareil de traitement d'informations selon la revendication 5, l'unité de commande étant configurée pour générer des informations de quantité de déviation pour présenter la quantité de déviation à un utilisateur sur la base de la quantité de déviation détectée.

**7.** Appareil de traitement d'informations selon la revendication 6,

l'unité de commande étant configurée pour générer les informations de quantité de déviation sur la base des informations concernant la partie marqueur.

8. Appareil de traitement d'informations selon la revendication 5,
l'unité de commande étant configurée pour modifier la surface tomographique sur la base de la quantité de déviation détectée.

9. Outil chirurgical (7), comprenant :

une partie de résine (12) comprenant une partie marqueur (15) prévue pour détecter une position ou un angle de rotation (Θ) autour d'un axe (X) le long d'une direction longitudinale d'un outil chirurgical dans une image tomographique d'un œil qui est soumis à une opération chirurgicale au moyen de l'outil chirurgical ;
la partie marqueur comprenant un motif géométrique particulier ; dans lequel
la partie marqueur comprend un premier marqueur (16) et un second marqueur (17), et le premier marqueur et le second marqueur sont agencés dans la partie de résine de telle sorte que lorsque l'outil chirurgical est tourné autour de l'axe le long d'une direction longitudinale de l'outil chirurgical, une distance entre le premier marqueur et le second marqueur dans la direction longitudinale varie en fonction de la rotation dans l'image tomographique le long de la direction longitudinale, et/ou un angle entre le premier marqueur et le second marqueur autour de l'axe varie en fonction de la rotation dans l'image tomographique le long d'une direction orthogonale à la direction longitudinale.

10. Procédé de traitement d'informations, comprenant :

l'acquisition, par une unité de commande (1), d'informations concernant une partie marqueur (15) représentée dans une image tomographique d'un œil qui est soumis à une opération chirurgicale au moyen d'un outil chirurgical (7) comprenant une partie de résine (12) comprenant la partie marqueur ; et
la partie marqueur comprenant un motif géométrique particulier ; et
la partie marqueur comprenant un premier marqueur (16) et un second marqueur (17), et
le premier marqueur et le second marqueur étant agencés dans la partie de résine de telle sorte que lorsque l'outil chirurgical est tourné autour d'un axe le long d'une direction longitudinale de l'outil chirurgical, une distance entre le premier marqueur et le second marqueur dans la direction longitudinale varie en fonction de la rotation dans une image tomographique le long de la direction longitudinale, et/ou un angle entre le premier marqueur et le second marqueur autour de l'axe varie en fonction de la rotation dans l'image tomographique le long d'une direction orthogonale à la direction longitudinale ;
la détection, par l'unité de commande, d'une position ou d'un angle de rotation (Θ) autour de l'axe (X), de l'outil chirurgical sur la base des informations concernant la partie marqueur.

11. Programme qui amène un ordinateur à exécuter :
un procédé selon la revendication 10.

10

1

| 2 — Front image acquisition unit | | Control unit | Storage unit | — 4 |
| 3 — Tomographic image acquisition unit | | | Display unit | — 5 |
| | | | Input unit | — 6 |

# FIG.1

FIG.2

FIG.3

7

11                                    12        13

15(15a)

Y'

θ

Z'    X'

FIG.4

FIG.5

FIG.6

FIG.7

FIG.8

FIG.9

FIG.10

FIG.11

START

Acquire planar image —ST101

Detect distal end position of surgical tool —ST102

ST103
Detect? — N / Y

Set measurement region so as to include distal end of surgical tool —ST104

Acquire tomographic image —ST105

Detect marker portion —ST106

ST107
Detect? — N / Y

Generate position or attitude information of surgical tool —ST108

Superimpose position or attitude information on planar image —ST109

Superimpose position or attitude information on tomographic image —ST110

Display planar image and tomographic image —ST111

ST112
Termination instruction? — N / Y

END

FIG.12

FIG.13

FIG.14

FIG.15

FIG.16

START

ST201
Is tomographic
surface input by user?
N

Y

Acquire tomographic
image
ST202

Detect marker portion
ST203

ST204
Detect?
N

Y

Calculate
amount-of-deviation
ST205

Generate
amount-of-deviation information
ST206

Present
amount-of-deviation information
ST207

ST208
Termination instruction?
N

Y

END

FIG.17

FIG.18

FIG.19

EP 3 603 484 B1

FIG.20

33

FIG.21

12

33

q'₁

p'₁

q'₂

p'₂

33

Z'

θ

Y'  X'

# FIG.22

12

q₁

33

p₁

q₂

p₂

33

Z'

θ

Y'  X'

# FIG.23

**EP 3 603 484 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014221822 A1

**Non-patent literature cited in the description**

- **EHLERS, JUSTIS P. et al.** Integrative advances for OCT-guided ophthalmic surgery and intraoperative OCT: microscope integration, surgical instrumentation, and heads-up display surgeon feedback. *PLoS One,* 2014, vol. 9 (8), e105224